(19) Europäisches Patentamt
European Patent Office
Office européen des brevets

(11) Veröffentlichungsnummer: **0 095 117**
**A1**

# (12) EUROPÄISCHE PATENTANMELDUNG

(21) Anmeldenummer: 83104792.3

(22) Anmeldetag: 16.05.83

(51) Int. Cl.³: C 07 C 59/68
C 07 C 69/712, C 07 C 149/14
C 07 C 51/00, C 07 C 67/00
C 07 C 43/295, C 07 C 41/16
C 07 C 153/07, A 01 N 39/04

(30) Priorität: 26.05.82 DE 3219789

(43) Veröffentlichungstag der Anmeldung:
30.11.83 Patentblatt 83/48

(84) Benannte Vertragsstaaten:
AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Förster, Heinz, Dr.
Am Eckbusch 47
D-5600 Wuppertal 1(DE)

(72) Erfinder: Marhold, Albrecht, Dr.
Carl-Duisberg-Strasse 329
D-5090 Leverkusen 1(DE)

(72) Erfinder: Priesnitz, Uwe, Dr.
Severinstrasse 58
D-5650 Solingen(DE)

(72) Erfinder: Riebel, Hans-Jochem, Dr.
In der Beek 92
D-5600 Wuppertal 1(DE)

(72) Erfinder: Eue, Ludwig, Dr.
Paul-Klee-Strasse 36
D-5090 Leverkusen 1(DE)

(72) Erfinder: Schmidt, Robert R., Dr.
Im Waldwinkel 110
D-5060 Bergisch Gladbach 2(DE)

## (54) Phenoxypropionsäure-Derivate, Verfahren zu ihrer Herstellung und ihre Verwendung als Herbizide.

(57) Phenoxypropionsäure-Derivative der Formel

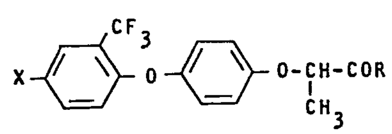

(I)

in welcher
R für Hydroxy, Chlor, Alkoxy, Halogenalkoxy, Alkoxyalkoxy, Alkenoxy, Alkinoxy, Aralkoxy, Alkylthio oder Alkylthioalkoxy steht und
X für Wasserstoff oder Halogen steht,
mehrere Verfahren zur Herstellung der Stoffe der Formel (I) und deren Verwendung zur Bekämpfung von Unkräutern.

Diphenylether der Formel

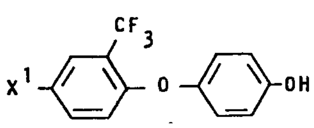

(IIa)

in welcher
X¹ für Wasserstoff, Fluor oder Brom steht,
und ein Verfahren zu deren Herstellung.

EP 0 095 117 A1

Croydon Printing Company Ltd.

BAYER AKTIENGESELLSCHAFT 5090 Leverkusen, Bayerwerk

Zentralbereich
Patente, Marken und Lizenzen Dü/Kü-c
Ib

# Phenoxypropionsäure-Derivate, Verfahren zu ihrer Herstellung und ihre Verwendung als Herbizide

Die Erfindung betrifft neue Phenoxypropionsäure-Derivate, mehrere Verfahren zu ihrer Herstellung sowie ihre Verwendung als Herbizide.

Es ist bereits bekannt geworden, daß zahlreiche Phenoxypropionsäure-Derivate herbizide Eigenschaften besitzen (vgl. DE-OS 2 223 894). So kann zum Beispiel der 2-/4-(2,4-Dichlorphenoxy)-phenoxy7-propionsäure-methylester zur Unkrautbekämpfung eingesetzt werden. Die Wirkung dieses Stoffes ist jedoch insbesondere bei einigen Gräsern und bei der Anwendung niedriger Konzentrationen nicht immer ausreichend.

Es wurden nun neue Phenoxypropionsäure-Derivate der Formel

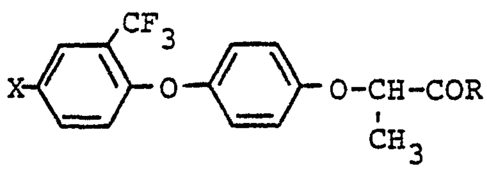

(I)

in welcher

R für Hydroxy, Chlor, Alkoxy, Halogenalkoxy, Alkoxyalkoxy, Alkenoxy, Alkinoxy, Aralkoxy, Alkylthio oder Alkylthioalkoxy steht und

X für Wasserstoff oder Halogen steht,

gefunden.

Die Phenoxypropionsäure-Derivate der Formel (I) enthalten ein asymmetrisches Kohlenstoffatom im Säureteil und können deshalb in zwei enantiomeren Formen vorliegen. Die Erfindung betrifft sowohl die jeweiligen Racemate als auch die R- und S-Enantiomeren.

Weiterhin wurde gefunden, daß man Phenoxypropionsäure-Derivate der Formel (I) erhält, wenn man

a) Diphenylether der Formel

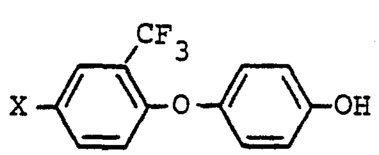

(II)

in welcher

X die oben angegebene Bedeutung hat

mit Propionsäure-Derivaten der Formel

$$Y - \underset{CH_3}{\underset{|}{CH}} - COR^1 \qquad (III)$$

in welcher

$R^1$ für Alkoxy, Halogenalkoxy, Alkoxyalkoxy, Alkenoxy, Alkinoxy, Aralkoxy, Alkylthio oder Alkylthioalkoxy steht, und

Y für Halogen, Tosylat oder Mesylat steht,

gegebenenfalls in Gegenwart eines Säureakzeptors und gegebenenfalls in Gegenwart eines Verdünnungsmittels umsetzt,

oder

b) Phenoxypropionsäure-Derivate der Formel

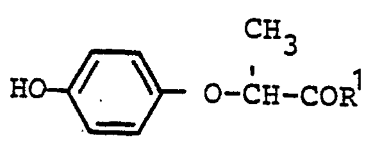

(IV)

in welcher

$R^1$ die oben angegebene Bedeutung hat,

mit Trifluormethylbenzol-Derivaten der Formel

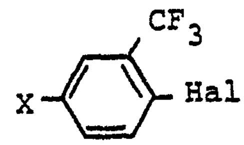

(V)

in welcher

X die oben angegebene Bedeutung hat und

Hal für Chlor oder Brom steht,

gegebenenfalls in Gegenwart eines Säureakzeptors und gegebenenfalls in Gegenwart eines Verdünnungsmittels umsetzt,

oder

c) Phenoxypropionsäure-Derivate der Formel

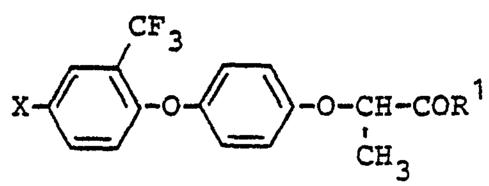

(Ia)

in welcher

$R^1$ und X die oben angegebene Bedeutung haben,

mit starken Basen in Gegenwart eines Verdünnungsmittels behandelt und anschließend ansäuert,

oder

d) Phenoxypropionsäure-Derivate der Formel

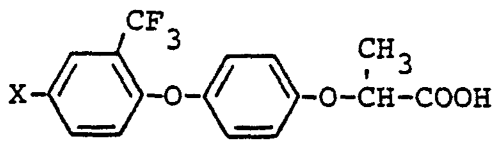

(Ib)

in welcher

X die oben angegebene Bedeutung hat,

mit Thionylchlorid gegebenenfalls in Gegenwart eines Verdünnungsmittels sowie gegebenenfalls in Gegenwart eines Katalysators umsetzt,

oder

e) Phenoxypropionsäure-Derivate der Formel

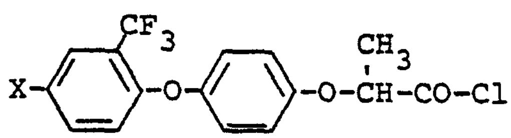

(Ic)

in welcher

X die oben angegebene Bedeutung hat,

mit Verbindungen der Formel

$$MR^1 \qquad (VI)$$

in welcher

$R^1$ die oben angegebene Bedeutung hat und

M für Wasserstoff, Natrium oder Kalium steht,

gegebenenfalls in Gegenwart eines Säurebindemittels und gegebenenfalls in Gegenwart eines Verdünnungsmittels umsetzt.

Schließlich wurde gefunden, daß sich die neuen Phenoxypropionsäure-Derivate der Formel (I) durch eine hervorragende herbizide Wirksamkeit auszeichnen.

Überraschenderweise besitzen die erfindungsgemäßen Phenoxypropionsäure-Derivate der Formel (I) wesentlich bessere herbizide Eigenschaften als der aus dem Stand der Technik bekannte 2-[4-(2,4-Dichlorphenoxy)-phenoxy]-propionsäure-methylester, welches ein hoch wirksamer Wirkstoff gleicher Wirkungsart ist. Vor allem lassen sich mit Hilfe der erfindungsgemäßen Wirkstoffe einige Ungräser, die von dem 2-[4-(2,4-Dichlorphenoxy)-phenoxy]-propionsäure-methylester nicht vollerfaßt werden, wirksam bekämpfen.

Die erfindungsgemäßen Phenoxypropionsäure-Derivate sind durch die Formel (I) definiert. In dieser Formel steht R vorzugsweise für Hydroxy, Chlor, Alkoxy mit 1 bis 8 Kohlenstoffatomen, Halogenalkoxy mit 1 bis 8 Kohlenstoffatomen und 1 bis 5 Halogenatomen, insbesondere Fluor-, Chlor- und Bromatomen. R steht weiterhin vorzugsweise für

Alkoxyalkoxy mit 1 bis 6 Kohlenstoffatomen in jeder Alkoxygruppe, Alkenoxy mit 2 bis 6 Kohlenstoffatomen, Alkinoxy mit 2 bis 6 Kohlenstoffatomen, Aralkoxy mit 6 bis 10 Kohlenstoffatomen im Arylteil und 1 bis 4 Kohlenstoffatomen im Alkoxyteil, für Alkylthio mit 1 bis 6 Kohlenstoffatomen, sowie für Alkylthioalkoxy mit 1 bis 6 Kohlenstoffatomen in der Alkylthiogruppe und 1 bis 6 Kohlenstoffatomen im Alkoxyteil. X steht vorzugsweise für Wasserstoff, Fluor, Chlor und Brom.

Besonders bevorzugt sind diejenigen Verbindungen der Formel (I), in denen R für Hydroxy, Chlor, geradkettiges oder verzweigtes Alkoxy mit 1 bis 6 Kohlenstoffatomen, Halogenalkoxy mit 1 bis 4 Kohlenstoffatomen und 1 bis 3 Fluor-, Chlor- und/oder Bromatomen, Alkoxyalkoxy mit 1 bis 4 Kohlenstoffatomen in jeder Alkoxygruppe, Alkenoxy mit bis zu 4 Kohlenstoffatomen, Alkinoxy mit bis zu 4 Kohlenstoffatomen, Phenalkoxy oder Naphthalkoxy mit jeweils 1 bis 3 Kohlenstoffatomen im Alkoxyteil, für Alkylthio mit 1 bis 4 Kohlenstoffatomen oder für Alkylthioalkoxy mit 1 bis 4 Kohlenstoffatomen im Alkylthioteil und 1 bis 4 Kohlenstoffatomen im Alkoxyteil steht, und X für Wasserstoff, Fluor, Chlor oder Brom steht.

Als Beispiele für Phenoxypropionsäure-Derivate der Formel (I) seien die in der folgenden Tabelle formelmäßig aufgeführten Verbindungen genannt:

Tabelle 1

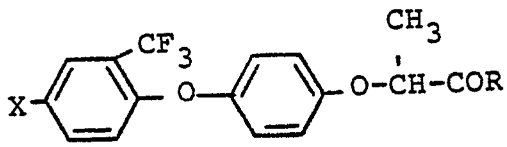

(I)

| X | R |
|---|---|
| Cl | $-OC_2H_5$ |
| Cl | $-OC_3H_7$-n |
| Cl | $-OC_3H_7$-iso |
| Cl | $-OC_4H_9$-n |
| Cl | $-OC_4H_9$-sek. |
| Cl | $-OC_4H_9$-iso |
| Cl | $-OC_4H_9$-tert. |
| Cl | $-OC_5H_{11}$-n |
| Cl | $-OCH_2-CH=CH_2$ |
| Cl | $-OCH_2-C\equiv CH$ |
| Cl | $-OCH_2-CH_2-OCH_3$ |
| Cl | $-OCH_2-CH_2-Cl$ |
| Cl | $-SCH_3$ |
| Cl | $-SC_2H_5$ |
| Cl | $-Cl$ |
| Cl | $-O-CH(CH_2Cl)_2$ |
| Cl | 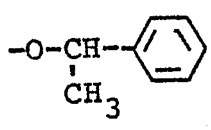 |
| Cl | 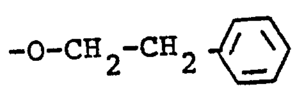 |

Tabelle 1 (Fortsetzung)

| X | R |
|---|---|
| Cl | $-O-CH_2-CCl_3$ |
| Cl | $-O-CH_2-CF_3$ |
| F | $-OC_2H_5$ |
| F | $-OC_3H_7$-n |
| F | $-OC_3H_7$-iso |
| F | $-OC_4H_9$-n |
| F | $-OC_4H_9$-sek. |
| F | $-OC_4H_9$-iso |
| F | $-OC_4H_9$-tert. |
| F | $-OC_5H_{11}$-n |
| F | $-OCH_2-CH=CH_2$ |
| F | $-OCH_2-C\equiv CH$ |
| F | 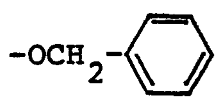 |
| F | $-OCH_2-CH_2-OCH_3$ |
| F | $-OCH_2-CH_2-Cl$ |
| F | $-SCH_3$ |
| F | $-SC_2H_5$ |
| F | -Cl |
| F | $-O-CH(CH_2Cl)_2$ |
| F | 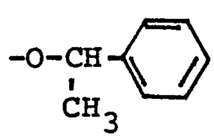 |

Tabelle 1 (Fortsetzung)

| X | R |
|---|---|
| F | $-O-CH_2-CH_2-$ |
| F | $-O-CH_2-CCl_3$ |
| F | $-O-CH_2-CF_3$ |
| F | $-O-CH_2-CH_2-S-C_2H_5$ |
| F | $-O-CH-C{\equiv}CH$ <br> $\quad\vert$ <br> $\quad CH_3$ |
| H | $-OC_2H_5$ |
| H | $-OC_3H_7$-n |
| H | $-OC_3H_7$-iso |
| H | $-OC_4H_9$-n |
| H | $-OC_4H_9$-sek. |
| H | $-OC_4H_9$-iso |
| H | $-OC_4H_9$-tert. |
| H | $-OC_5H_{11}$-n |
| H | $-OCH_2-CH=CH_2$ |
| H | $-OCH_2-C{\equiv}CH$ |
| H | $-OCH_2-CH_2-OCH_3$ |
| H | $-OCH_2-CH_2-Cl$ |
| H | $-SCH_3$ |
| H | $-SC_2H_5$ |

Tabelle 1 (Fortsetzung)

| X | R |
|---|---|
| H | -Cl |
| H | $-O-CH(CH_2Cl)_2$ |
| H | 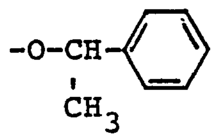 |
| H | 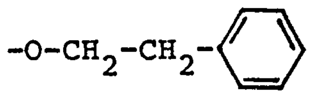 |
| H | $-O-CH_2-CCl_3$ |
| H | $-O-CH_2-CF_3$ |
| H | $-O-CH_2-CH_2-S-C_2H_5$ |

Besonders bevorzugt sind auch die R-Enantiomeren der zuvor genannten Verbindungen.

Verwendet man 4-Chlor-2-trifluormethyl-4'-hydroxy-diphenylether und 2-Brom-propionsäure-ethylester als Ausgangsstoffe, so kann der Verlauf des erfindungsgemäßen Verfahrens (a) durch das folgende Formelschema wiedergegeben werden:

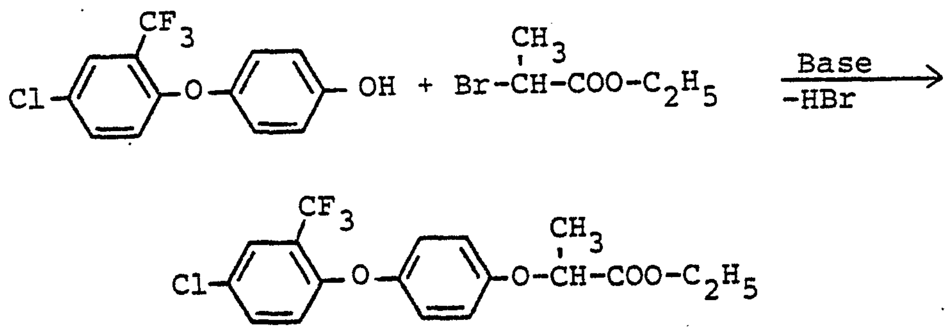


Verwendet man 2-(4-Hydroxy-phenoxy)-propionsäure-n-butylester und 4-Brom-3-trifluormethyl-chlorbenzol als Ausgangsstoffe, so kann der Verlauf des erfindungsgemäßen Verfahrens (b) durch das folgende Formelschema wiedergegeben werden:

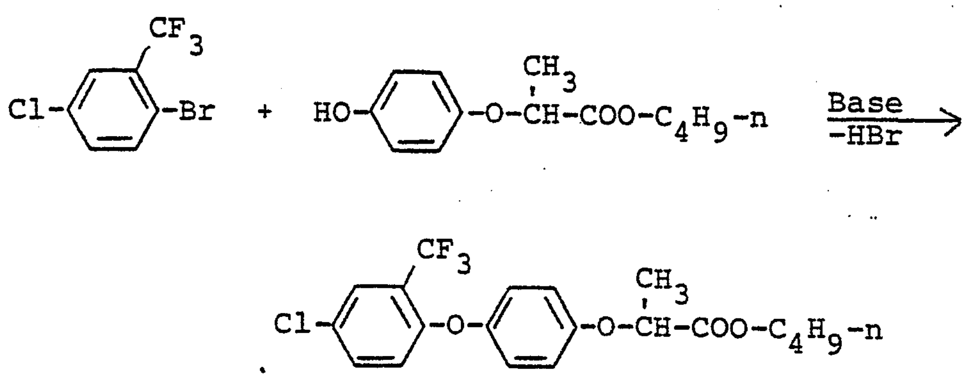

Verwendet man 2-[4-(4-Chlor-2-trifluormethyl-phenoxy)-phenoxy]-propionsäure-ethylester als Ausgangsstoff, Natriumhydroxid als Base und Chlorwasserstoff als Säure, so kann der Verlauf des erfindungsgemäßen Verfahrens (c) durch das folgende Formelschema wiedergegeben werden:

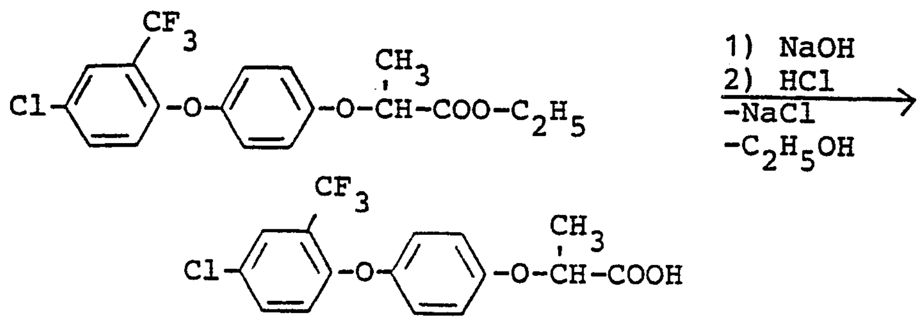


Verwendet man 2-[4-(4-Chlor-2-trifluormethyl-phenoxy)-phenoxy]-propionsäure als Ausgangsstoff und Thionylchlorid als Reaktionskomponente, so kann der Verlauf des erfindungsgemäßen Verfahrens (d) durch das folgende Formelschema wiedergegeben werden:

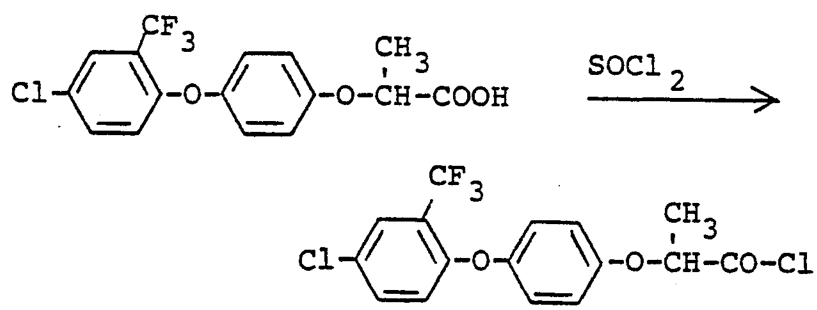

Verwendet man 2-/4-(4-Chlor-2-trifluormethyl-phenoxy)-phenoxy7-propionsäure-chlorid und n-Propanol als Ausgangsstoffe, so kann der Verlauf des erfindungsgemäßen Verfahrens (e) durch das folgende Formelschema wiedergegeben werden:

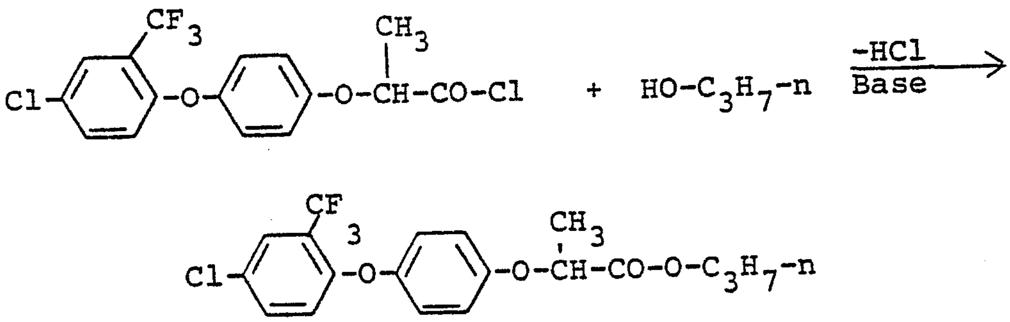


Die bei dem erfindungsgemäßen Verfahren (a) als Ausgangsstoffe benötigten Diphenylether sind durch die Formel (II) eindeutig definiert. In dieser Formel steht X vorzugsweise für Wasserstoff, Fluor, Chlor und Brom.

Als Beispiele für Diphenylether der Formel (II) seien genannt: 4-Chlor-2-trifluormethyl-4'-hydroxy-diphenylether, 4-Brom-2-trifluormethyl-4'-hydroxy-diphenylether, 4-Fluor-2-trifluormethyl-4'-hydroxy-diphenylether und 2-Trifluormethyl-4'-hydroxy-diphenylether.

Von den Diphenylethern der Formel (II) ist bisher nur der 4-Chlor-2-trifluormethyl-4'-hydroxy-diphenylether bekannt (vgl. DE-OS 2 538 016). Die Stoffe der Formel

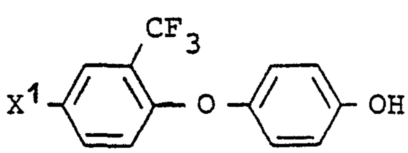 (IIa)

in welcher

$X^1$ für Wasserstoff, Fluor oder Brom steht,

sind neu. Sie lassen sich herstellen, indem man Trifluormethylbenzol-Derivate der Formel

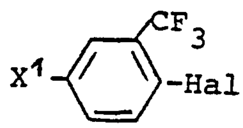

(Va)

in welcher

$X^1$ und Hal die oben angegebene Bedeutung haben,

mit Hydrochinon in Gegenwart eines Säureakzeptors, wie zum Beispiel Calciumhydroxid, sowie in Gegenwart eines polaren Verdünnungsmittels, wie zum Beispiel Acetonitril, Dimethylformamid oder Dimethylsulfoxid, bei Temperaturen zwischen 20 und 200°C, vorzugsweise zwischen 50 und 140°C, umsetzt.

Die bei dem erfindungsgemäßen Verfahren (a) weiterhin als Ausgangsstoffe benötigten Propionsäure-Derivate sind durch die Formel (III) eindeutig definiert. In dieser Formel steht $R^1$ vorzugsweise für Alkoxy mit 1 bis 8 Kohlenstoffatomen, Halogenalkoxy mit 1 bis 8 Kohlenstoffatomen und 1 bis 5 Halogenatomen, insbesondere Fluor-, Chlor- und Bromatomen. $R^1$ steht weiterhin vorzugsweise für Alkoxyalkoxy mit 1 bis 6 Kohlenstoff-

atomen in jeder Alkoxygruppe, Alkenoxy mit 2 bis 6 Kohlenstoffatomen, Alkinoxy mit 2 bis 6 Kohlenstoffatomen, Aralkoxy mit 6 bis 10 Kohlenstoffatomen im Arylteil und 1 bis 4 Kohlenstoffatomen im Alkoxyteil, für Alkylthio mit 1 bis 6 Kohlenstoffatomen sowie für Alkylthioalkoxy mit 1 bis 6 Kohlenstoffatomen in der Alkylthiogruppe und 1 bis 6 Kohlenstoffatomen im Alkoxyteil. Y steht vorzugsweise für Chlor, Brom, Mesylat oder Tosylat.

Als Beispiele für Propionsäure-Derivate der Formel (III) seien die in der folgenden Tabelle formelmäßig aufgeführten Verbindungen genannt:

Tabelle 2

$$Y - \overset{CH_3}{\overset{|}{C}H} - COR^1 \qquad (III)$$

| Y | $R^1$ | Y | $R^1$ |
|---|---|---|---|
| | | Cl | $-OCH_2-C\equiv CH$ |
| Cl | $OC_2H_5$ | Cl | $-OCH_2-$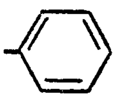 |
| Cl | $OC_3H_7$-n | Cl | $-OCH_2-CH_2-OCH_3$ |
| Cl | $OC_3H_7$-iso | Cl | $-OCH_2-CH_2-Cl$ |
| Cl | $OC_4H_9$-n | Cl | $-SCH_3$ |
| Cl | $OC_4H_9$-sek. | Cl | $-SC_2H_5$ |
| Cl | $OC_4H_9$-tert. | | |
| Cl | $OC_5H_{11}$-n | Br | $OC_2H_5$ |
| Cl | $-OCH_3-CH=CH_2$ | Br | $OC_3H_7$-n |
| Br | $OC_3H_7$-iso | MesO | $OCH_2-$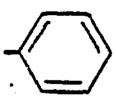 |
| Br | $OC_4H_9$-n | MesO | $OCH_2-CH_2-OCH_3$ |
| Br | $OC_4H_9$-sek. | MesO | $OCH_2-CH_2-Cl$ |
| Br | $OC_4H_9$-iso | MesO | $SCH_3$ |
| Br | $OC_4H_9$-tert. | MesO | $SC_2H_5$ |
| Br | $OC_5H_{11}$-n | TosO | $OC_2H_5$ |
| Br | $OCH_2-CH=CH_2$ | TosO | $OC_3H_7$-n |
| Br | $OCH_2-C=CH$ | TosO | $OC_3H_7$-iso |
| Br | $OCH_2-$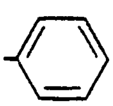 | TosO | $OC_4H_9$-n |
| Br | $OCH_2-CH_2-OCH_3$ | TosO | $OC_4H_9$-sek. |
| Br | $OCH_2-CH_2-Cl$ | TosO | $OC_4H_9$-iso |
| Br | $SCH_3$ | TosO | $OC_4H_9$-tert. |

<u>Tabelle 2</u> (Fortsetzung)

| Y | $R^1$ | Y | $R^1$ |
|---|---|---|---|
| Br | $SC_2H_5$ | TosO | $OC_5H_{11}$-n |
| | | TosO | $OCH_2-CH=CH_2$ |
| MesO | $OC_2H_5$ | TosO | $OCH_2-C\equiv CH$ |
| MesO | $OC_3H_7$-n | TosO | $OCH_2$- 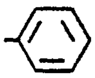 |
| MesO | $OC_3H_7$-iso | TosO | $OCH_2-CH_2-OCH_3$ |
| MesO | $OC_4H_9$-n | TosO | $OCH_2-CH_2-Cl$ |
| MesO | $OC_4H_9$-sek. | TosO | $SCH_3$ |
| MesO | $OC_4H_9$-iso | TosO | $SC_2H_5$ |
| MesO | $OC_4H_9$-tert. | Br | $O-CH(CH_2Cl)_2$ |
| MesO | $OC_5H_{11}$-n | Br | $O-CH$- 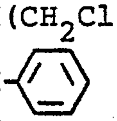<br>$CH_3$ |
| MesO | $OCH_2-CH=CH_2$ | | |
| MesO | $OCH_2-C\equiv CH$ | Br | $O-CH_2-CH_2$- 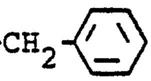 |
| Br | $O-CH_2-Cl_3$ | | |
| Br | $O-CH_2-CF_3$ | | |
| Br | $O-CH_2-CH_2-S-C_2H_5$ | | |
| Br | $O-CH-C\equiv CH$<br>$CH_3$ | | |

TosO = Tosylat
MesO = Mesylat

Die Propionsäure-Derivate der Formel (III) sind bekannt oder lassen sich nach bekannten Verfahren in einfacher Weise herstellen.

Zur Herstellung der R- und der S-Enantiomeren der Phenoxypropionsäure-Derivate der Formel (I) werden optisch aktive Propionsäure-Derivate der Formel (III) bei der Durchführung des erfindungsgemäßen Verfahrens (a) als Ausgangsstoffe benötigt. Auch diese optisch aktiven Propionsäure-Derivate der Formel (III) sind bekannt oder lassen sich nach bekannten Racemat-Spaltungsmethoden herstellen.

Man erhält R-enantiomere bzw. S-enantiomere Phenoxypropionsäure-Derivate der Formel (I), indem man bei der Durchführung des erfindungsgemäßen Verfahrens (a) optisch aktive S-Enantiomere bzw. R-Enantiomere der Propionsäure-Derivate der Formel

$$Y-\underset{\displaystyle CH_3}{\overset{*}{C}}H-COR^1 \qquad (III)$$

in welcher

$R^1$ und Y die oben angegebene Bedeutung haben,

einsetzt.

Bei dieser Umsetzung findet eine Walden-Umkehr statt. Dieses hat zur Folge, daß durch Umsetzung von Diphenylethern der Formel (II) mit den S-Enantiomeren der Propionsäure-Derivate der Formel (III) die jeweiligen R-Enantiomeren der Phenoxypropionsäure-Derivate der Formel (I) entstehen. Andererseits bilden sich durch Umsetzung von

Diphenylethern der Formel (II) mit den R-Enantiomeren der Propionsäure-Derivate der Formel (III) die jeweiligen S-Enantiomeren der Phenoxypropionsäure-Derivate der Formel (I).

Die bei dem erfindungsgemäßen Verfahren (b) als Ausgangsstoffe benötigten Phenoxypropionsäure-Derivate sind durch die Formel (IV) definiert. In dieser Formel steht $R^1$ vorzugsweise für diejenigen Reste, die bereits im Zusammenhang mit der Beschreibung der Propionsäure-Derivate der Formel (III) vorzugsweise für diesen Rest genannt wurden.

Als Beispiele für Phenoxypropionsäure-Derivate der Formel (IV) seien die in der folgenden Tabelle formelmäßig aufgeführten Verbindungen genannt:

Tabelle 3

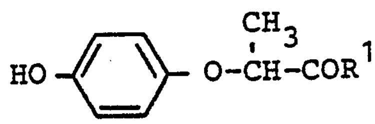

(IV)

| $R^1$ | $R^1$ |
|---|---|
| $-OC_2H_5$ | 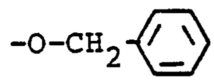 |
| $-OC_3H_7$-n | $-O-CH_2-CH_2-OCH_3$ |
| $-OC_3H_7$-iso | $-O-CH_2-CH_2-Cl$ |
| $-OC_4H_9$-n | $-SCH_3$ |
| $-OC_4H_9$-sek. | $-SC_2H_5$ |
| $-O-CH(CH_2Cl)_2$ | $-O-CH_2-CF_3$ |
| 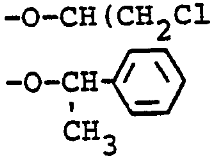 | $-O-CH_2-CH_2-S-C_2H_5$ |
| 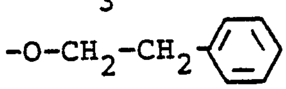 | $-O-CH(CH_3)-C\equiv CH$ |
| $-O-CH_2-CCl_3$ | |
| $-OC_4H_9$-tert. | |
| $-OC_5H_{11}$-n | |
| $-OCH_2-CH=CH_2$ | |
| $-O-CH_2-C\equiv CH$ | |

Die Phenoxypropionsäure-Derivate der Formel (IV) sind bekannt oder lassen sich nach bekannten Verfahren in einfacher Weise herstellen (vgl. DE-OS 2 639 796). So erhält man die Verbindungen der Formel (IV) zum Beispiel dadurch, daß man Hydrochinon mit einem Propionsäure-Derivat der Formel

$$Y - \underset{\displaystyle CH_3}{\underset{|}{CH}} - COR^1 \qquad \text{(III)}$$

in welcher

Y und $R^1$ die oben angegebene Bedeutung haben,

in Gegenwart eines Säurebindemittels und in Gegenwart eines Verdünnungsmittels bei Temperaturen zwischen 20°C und 120°C umsetzt.

Die bei dem erfindungsgemäßen Verfahren (b) weiterhin als Ausgangsstoffe benötigten Trifluormethylbenzol-Derivate sind durch die Formel (V) eindeutig definiert. In dieser Formel steht X vorzugsweise für Wasserstoff, Fluor, Chlor und Brom. Hal steht für Chlor oder Brom.

Als Beispiele für Verbindungen der Formel (V) seien genannt: 4-Brom-2-trifluormethyl-chlorbenzol, 4-Brom-3-trifluormethyl-fluorbenzol, 1,4-Dibrom-2-trifluormethylbenzol, 1,4-Dichlor-2-trifluormethyl-benzol, 1-Chlor-4-fluor-2-trifluormethyl-benzol, 1-Chlor-2-trifluormethyl-benzol und 1-Brom-2-trifluormethyl-benzol.

Die Verbindungen der Formel (V) sind bekannt oder lassen sich nach bekannten Verfahren in einfacher Weise herstellen (vgl. DE-OS 2 732 442). So erhält man Verbindungen der Formel (V) zum Beispiel dadurch, daß man

α) Anilin-Derivate der Formel

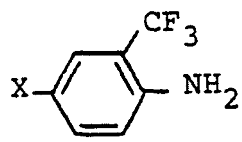

(VII)

in welcher

X die oben angegebene Bedeutung hat,

zunächst mit Natriumnitrit in Gegenwart von Bromwasserstoffsäure im wäßrigen Medium bei Temperaturen zwischen -10°C und +5°C diazotiert und anschließend mit Bromwasserstoff gegebenenfalls in Gegenwart eines Katalysators, wie z.B. Kupferbromid, bei Temperaturen zwischen 20°C und 80°C umsetzt,

oder

ß) Trifluormethylbenzol-Derivate der Formel

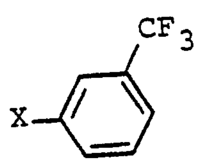

(VIII)

in welcher

X die oben angegebene Bedeutung hat,

mit Chlor oder Brom in Gegenwart eines Katalysators, wie zum Beispiel Eisen-(III)-chlorid, oder Eisen-(III)-bromid, bei Temperaturen zwischen 20°C und 100°C umsetzt,

oder

γ) Trichlormethylbenzol-Derivate der Formel

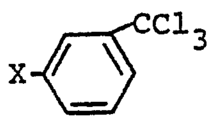

(IX)

in welcher

X die oben angegebene Bedeutung hat,

mit Brom oder Chlor in Gegenwart eines Katalysators, wie zum Beispiel Eisen-(III)-sulfid, bei Temperaturen zwischen 20°C und 100°C umsetzt und die dabei anfallenden Verbindungen der Formel

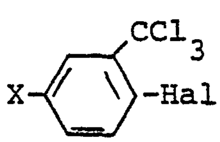

(X)

in welcher

X und Hal die oben angegebene Bedeutung haben,

mit Fluorwasserstoff bei Temperaturen zwischen 40°C und 100°C gegebenenfalls unter Druck umsetzt,

oder

δ) Brombenzol der Formel

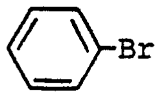

(XI)

mit Tetrachlorkohlenstoff in Gegenwart von Fluorwasserstoff bei Temperaturen zwischen 80°C und 150°C, vorzugsweise zwischen 90°C und 120°C, umsetzt.

Die bei diesen Umsetzungen als Ausgangsstoffe benötigten Verbindungen der Formeln (VII), (VIII), (IX) und (XI) sind bekannt oder lassen sich nach üblichen Verfahren in einfacher Weise herstellen.

Um R- und S-Enantiomere der Phenoxypropionsäure-Derivate der Formel (I) nach dem erfindungsgemäßen Verfahren (b) herzustellen, ist es erforderlich, die jeweiligen optisch aktiven Phenoxypropionsäure-Derivate der Formel (IV) als Ausgangsstoffe zu verwenden. Auch diese optisch aktiven Phenoxypropionsäure-Derivate der Formel (IV) sind bekannt oder lassen sich durch Umsetzung von Hydrochinon mit optisch aktiven Propionsäure-Derivaten der Formel (III) nach bekannten Methoden herstellen (vgl. Angaben zu Verfahren (a)).

Die bei der Durchführung des erfindungsgemäßen Verfahrens (c) als Ausgangsstoffe benötigten Phenoxypropionsäure-Derivate sind durch die Formel (Ia) definiert. In dieser Formel steht $R^1$ vorzugsweise für diejenigen Reste, die bereits im Zusammenhang mit der Beschreibung der Propionsäure-Derivate der Formel (III) vorzugsweise für diesen Rest genannt wurden. X steht vorzugsweise für Wasserstoff, Fluor, Chlor und Brom.

Die Verbindungen der Formel (Ia) sind bisher noch nicht bekannt. Sie lassen sich jedoch nach dem erfindungsgemäßen Verfahren (a) in einfacher Weise herstellen.

Als starke Basen, die im Falle des erfindungsgemäßen Verfahrens (c) als Reaktionskomponenten benötigt werden, kommen vorzugsweise Alkali- und Erdalkalihydroxid in Betracht. Als Beispiele seien Natriumhydroxid, Kaliumhydroxid und Calciumhydroxid genannt.

Als Säuren, die im Falle des erfindungsgemäßen Verfahrens (c) zur Freisetzung der Säuren der Formel (I) benötigt werden, kommen vorzugsweise starke anorganische Säuren in Frage. Als Beispiel genannt sei Salzsäure.

Um R- und S-Enantiomere der Phenoxypropionsäuren der Formel (I) nach dem erfindungsgemäßen Verfahren (c) herzustellen, ist es erforderlich, die jeweiligen optisch aktiven Phenoxypropionsäure-Derivate der Formel (Ia) als Ausgangsstoffe zu verwenden.

Die bei der Durchführung des erfindungsgemäßen Verfahrens (d) als Ausgangsstoffe benötigten Phenoxypropionsäure-Derivate sind durch die Formel (Ib) definiert. In dieser Formel steht X vorzugsweise für Wasserstoff, Fluor, Chlor und Brom.

Die Verbindungen der Formel (Ib) sind bisher noch nicht bekannt. Sie lassen sich jedoch nach dem erfindungsgemäßen Verfahren (c) in einfacher Weise herstellen.

Um R- und S-Enantiomere der Phenoxypropionsäure-chloride der Formel (I) nach dem erfindungsgemäßen Verfahren (d) herzustellen, ist es erforderlich, die jeweiligen optisch aktiven Phenoxypropionsäuren der Formel (Ib) als Ausgangsstoffe zu verwenden.

Die bei der Durchführung des erfindungsgemäßen Verfahrens (e) als Ausgangsstoffe benötigten Phenoxypropionsäure-Derivate sind durch die Formel (Ic) definiert. In dieser Formel steht X vorzugsweise für Wasserstoff, Fluor, Chlor und Brom.

Die Verbindungen der Formel (Ic) sind bisher noch nicht bekannt. Sie lassen sich jedoch nach dem erfindungsgemäßen Verfahren (d) in einfacher Weise herstellen.

Um R- und S-Enantiomere der Phenoxypropionsäure-Derivate der Formel (I) nach dem erfindungsgemäßen Verfahren (e) herzustellen, ist es erforderlich, die jeweiligen optisch aktiven Phenoxypropionsäure-chloride der Formel (Ic) als Ausgangsstoffe zu verwenden.

Die bei dem erfindungsgemäßen Verfahren (e) weiterhin als Ausgangsstoffe benötigten Verbindungen sind durch die Formel (VI) definiert. In dieser Formel steht $R^1$ vorzugsweise für diejenigen Reste, die bereits im Zusammenhang mit der Beschreibung der Propionsäure-Derivate der Formel (III) vorzugsweise für diesen Rest genannt wurden. M steht für Wasserstoff, Natrium oder Kalium.

Die Verbindungen der Formel (VI) sind bekannt oder lassen sich nach bekannten Methoden in einfacher Weise herstellen.

Die erfindungsgemäßen Verfahren (a) und (b) zur Herstellung der neuen Stoffe der Formel (I) werden vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl-, und Methyl-isobutyl-keton, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Als Säureakzeptoren können sowohl bei dem Verfahren (a) als auch bei dem Verfahren (b) alle üblicherweise für derartige Umsetzungen verwendbaren Säurebindemittel eingesetzt werden. Vorzugsweise in Frage kommen Alkalihydroxide, Erdalkalihydroxide und -oxide, wie z.B. Natrium- und Kaliumhydroxid, Calciumhydroxid und Calciumoxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzyl-amin, 1,5-Diazabicyclo/4,3,0/non-5-en (DBN), 1,8-Diazabicyclo/5,4,0/undec-7-en (DBU) und Pyridin.

Die Reaktionstemperaturen können sowohl bei dem erfindungsgemäßen Verfahren (a) als auch bei dem Verfahren (b) innerhalb eines größeren Bereiches variiert werden. Im allgemeinen arbeitet man jeweils bei Temperaturen zwischen -20°C und +150°C, vorzugsweise zwischen 0°C und 120°C.

Die erfindungsgemäßen Verfahren (a) und (b) werden im allgemeinen unter Normaldruck durchgeführt. Es ist jedoch auch möglich unter erhöhtem oder vermindertem Druck zu arbeiten.

Zur Durchführung des erfindungsgemäßen Verfahrens (a) werden die Ausgangsstoffe der Formeln (II) und (III) im allgemeinen in angenähert äquimolaren Mengen eingesetzt. Es ist jedoch auch möglich, eine der beiden Komponenten

in einem größeren Überschuß einzusetzen. Die Reaktion wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die Aufarbeitung erfolgt nach üblichen Methoden. Im allgemeinen geht man so vor, daß man das Reaktionsgemisch in Wasser gießt, das entstehende Gemisch mit einem organischen, in Wasser wenig löslichen Solvens extrahiert, die organische Phase trocknet und einengt.

Bei der Herstellung von optischen Isomeren der Phenoxypropionsäure-Derivate der Formel (I) arbeitet man zweckmäßigerweise nach der in der DE-OS 2 758 002 beschriebenen Methode.

Auch bei der Durchführung des erfindungsgemäßen Verfahrens (b) werden die Ausgangsstoffe im allgemeinen in etwa äquimolaren Mengen eingesetzt. Es ist jedoch auch möglich, eine der beiden Komponenten in einem größeren Überschuß einzusetzen. Die Reaktion wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die Aufarbeitung erfolgt auch bei diesem Verfahren nach üblichen Methoden.

Bei der Durchführung des erfindungsgemäßen Verfahrens (c) können als Verdünnungsmittel alle für derartige Verseifungen üblichen Solventien eingesetzt werden. Vorzugsweise in Frage kommen Alkohole, wie Methanol und Ethanol, ferner Kohlenwasserstoffe, wie Benzol, Toluol und Xylol, und außerdem Wasser, oder auch Gemische aus Wasser und Alkoholen bzw. Kohlenwasserstoffen.

Die Reaktionstemperaturen können bei dem erfindungsgemäßen Verfahren (c) innerhalb eines größeren Bereiches variiert werden. Im allgemeinen arbeitet man bei Temperaturen zwischen 20°C und 140°C, vorzugsweise 40°C und 120°C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) setzt man auf 1 Mol an Phenoxypropionsäure-Derivat der Formel (Ia) im allgemeinen 1 bis 3 Mol an Base ein. Die Isolierung der entstehenden Säuren erfolgt im allgemeinen dadurch, daß man das Reaktionsgemisch in Wasser gießt, ansäuert, mit einem mit Wasser wenig mischbaren organischen Lösungsmittel extrahiert, die organische Phase trocknet und einengt.

Bei der Durchführung des erfindungsgemäßen Verfahrens (d) können als Verdünnungsmittel alle für derartige Chlorierungen üblichen Solventien eingesetzt werden. Vorzugsweise in Betracht kommen gegebenenfalls chlorierte aliphatische oder aromatische Kohlenwasserstoffe, wie zum Beispiel Xylol, Toluol, Methylenchlorid und Ethylenchlorid.

Die Reaktionstemperaturen können bei dem erfindungsgemäßen Verfahren (d) innerhalb eines größeren Bereiches variiert werden. Im allgemeinen arbeitet man bei Temperaturen zwischen 0° und 50°C, vorzugsweise zwischen 10°C und 30°C.

Als Katalysatoren können bei dem erfindungsgemäßen Verfahren (d) alle für derartige Chlorierungen üblichen Reaktionsbeschleuniger eingesetzt werden. Beispielhaft genannt seien Dimethylformamid und Pyridin.

Das erfindungsgemäße Verfahren (d) wird im allgemeinen unter Normaldruck durchgeführt. Es ist jedoch auch möglich, unter erhötem oder vermindertem Druck zu arbeiten.

Zur Durchführung des erfindungsgemäßen Verfahrens (d) setzt man auf 1 Mol an Phenoxypropionsäure-Derivat der Formel (Ib) vorzugsweise 1 bis 1,5 Mol Thionylchlorid sowie gegebenenfalls eine katalytische Menge eines Reaktionsbeschleunigers ein. Die Aufarbeitung erfolgt nach üblichen Methoden.

Als Verdünnungsmittel kommen bei der Durchführung des erfindungsgemäßen Verfahrens (e) alle inerten organischen Solventien in Betracht. Vorzugsweise verwendbar sind aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl-, und Methyl-isobutyl-keton und Ester, wie Essigsäure-methylester und -ethylester. Wird als Reaktionskomponente der Formel (VI) ein Alkohol oder Mercaptan eingesetzt, so kann diese Verbindung gleichzeitig als Verdünnungsmittel fungieren, sofern sie in ausreichendem Überschuß verwendet wird.

Als Säureakzeptoren können bei dem erfindungsgemäßen Verfahren (e) alle üblichen Säurebindemittel eingesetzt werden. Vorzugsweise in Frage kommen alle diejenigen Stoffe, die bereits im Zusammenhang mit der Beschreibung der erfindungsgemäßen Verfahren (a) und (b) vorzugsweise als Säureakzeptoren genannt wurden.

Die Reaktionstemperaturen können auch bei dem erfindungsgemäßen Verfahren (e) innerhalb eines größeren Bereiches variiert werden. Im allgemeinen arbeitet man bei Temperaturen zwischen -20°C und +150°C, vorzugsweise zwischen 0°C und 120°C.

Das erfindungsgemäße Verfahren (e) wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens (e) setzt man auf 1 Mol an Phenoxypropionsäure-Derivat der Formel (Ic) 1 bis 2 Mol oder auch einen größeren

Überschuß an einer Verbindung der Formel (VI) sowie gegebenenfalls 1 bis 2 Mol an Säurebindemittel ein. Die Aufarbeitung erfolgt nach üblichen Methoden.

Die erfindungsgemäßen Wirkstoffe der Formel (I) können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungssgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können in dikotylen Kulturen sowie in Getreide und Reis zur selektiven Unkrautbekämpfung eingesetzt werden.

Die Wirkstoffe können in die üblichen Formulierungen übergeführt werden, wie Lösungen, Emulsionen, Spritzpulver, Suspensionen, Pulver, Stäubemittel, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage:
Aromaten, wie Xylol, Toluol, oder Alkylnaphthaline, chlorierte Aromaten oder chlorierte aliphatische Kohlenwasserstoffe, wie Chlorbenzol, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide,

Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkokohol, Polyvinylacetat.

Es können Farbstoffe wie anorganische Pigmente, z.B. Eisenoxid, Titanoxid, Ferrocyanblau und organische Farbstoffe, wie Alizarin-, Azofarbstoffe und Metallphthalocyaninfarbstoffe und Spurennährstoffe wie Salze von Eisen, Magan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4-(1H, 3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5 (4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Stoffe geht aus den nachfolgenden Beispielen hervor.

## Herstellungsbeispiele

### Beispiel 1

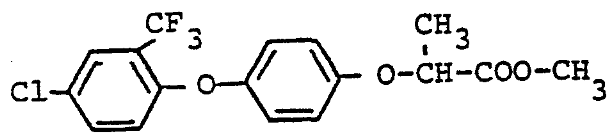

In eine Mischung aus 28,9 g (0,11 Mol) 4-(4-Chlor-2-trifluormethyl-phenoxy)-phenol und 7,6 g Kaliumcarbonat in 100 ml Acetonitril werden bei 60°C unter Rühren 16,7 g (0,1 Mol) α-Brom-propionsäure-methylester eingetropft. Es wird 6 Stunden unter Rückfluß nachgerührt. Danach läßt man abkühlen und arbeitet dann auf, indem man das Reaktionsgemisch auf Wasser gießt und das entstehende Gemisch mehrfach mit Methylenchlorid extrahiert. Die vereinigten organischen Phasen werden nacheinander mit verdünnter wäßriger Salzsäure und mit Wasser gewaschen, dann über Magnesiumsulfat getrocknet und durch Abziehen des Lösungsmittels eingeengt. Man erhält auf diese Weise 28,1 g (75 % der Theorie) an 2-[4-(4-Chlor-2-trifluormethyl-phenoxy)-phenoxy]-propionsäure-methylester in Form eines Öles.

$n_D^{19}$ = 1,5259.

Herstellung der Ausgangsstoffe:

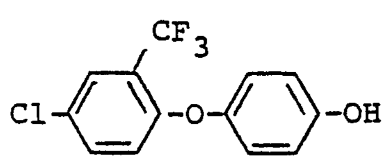

(II-1)

In eine Mischung aus 500 ml Dimethylsulfoxid, 132 g (1,2 Mol) Hydrochinon und 114 g gepulvertem Kaliumhydroxid wird bei einer Temperatur von 80°C bis 90°C unter Rühren innerhalb einer Stunde eine Lösung von 259,5 g (1 Mol) 2-Brom-5-Chlor-benzotrifluorid in 200 ml Dimethylsulfoxid getropft. Man rührt weitere 4 Stunden bei 80°C und zieht dann das Lösungsmittel ab. Der verbleibende Rückstand wird in 2 l Wasser gegossen, und die entstehende Mischung wird mit 100 ml konzentrierter wäßriger Salzsäure angesäuert. Das Gemisch wird zweimal mit je 1 l Chloroform extrahiert. Die vereinigten organischen Phasen werden zweimal mit einer Lösung von 50 ml konzentrierter wäßriger Natronlauge in 1 l Wasser ausgeschüttelt. Man säuert die vereinigten wäßrigen Phasen mit 150 ml konzentrierter wäßriger Salzsäure an und extrahiert das sich dabei abscheidende Öl zweimal mit je 1 l Chlorofom. Man engt die vereinigten organischen Phasen ein und erhält auf diese Weise 245 g (85 % der Theorie) an 4-(4-Chlor-2-trifluormethyl-phenoxy)-phenol in Form eines Öles.

$n_D^{20} = 1,546$

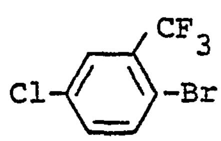

(VII-1)

(Variante α)

Man legt 500 ml einer 48 %-igen wäßrigen Bromwasserstoff-Lösung vor und tropft unter Rühren 195 g (1,03 Mol)

2-Trifluormethyl-4-chlor-anilin zu. Dann wird rasch auf 0°C abgekühlt und mit einer Lösung von 69 g (1 Mol) Natriumnitrit in 150 ml Wasser versetzt, wobei Diazotierung eintritt. In einem zweiten Reaktionskolben wird ein Gemisch aus 100 ml 48 %-iger wäßriger Bromwasserstoff-Lösung und 80 g Kupferbromid auf 100°C erhitzt. In dieses Gemisch wird die gekühlte Diazonium-Lösung unter Rühren eingetropft, wobei die Temperatur des Reaktionsgemisches auf etwa 100°C gehalten wird. Nach beendeter Umsetzung wird das entstehende Produkt mit Wasserdampf überdestilliert. Die organische Phase wird abgetrennt und erneut destilliert. Man erhält auf diese Weise 202 g (77,8 % der Theorie) an 2-Brom-5-chlor-benzotrifluorid.

Kp = 75 - 77°C / 16 mbar

$n_D^{20}$ = 1,5067

(Variante γ )

In eine Mischung aus 820 g (3,6 Mol) 3-Chlorbenzotrichlorid und 3 g Eisensulfid werden bei 50°C innerhalb von 4 Stunden 625 g (3,6 Mol) Brom unter Rühren eingetropft. Es wird weitere 4 Stunden bei 50°C gerührt und dann bei 15 mbar entgast. Die dabei verbleibenden 1160 g an Rohprodukt werden bei 0°C in 750 ml Fluorwasserstoff getropft, der sich in einer Fluorierungsapparatur befindet. Man rührt zunächst bei 15 bis 20°C bis zum Ende der Gasentwicklung und fluoriert anschließend unter erhöhtem Druck bei 70 bis 80°C zu Ende. Das anfallende Reaktionsgemisch wird destilliert. Man erhält auf diese Weise 538 g (58,2 % der Theorie) an 2-Brom-5-chlor-benzotrifluorid.

Beispiel 2

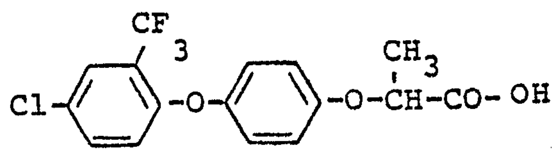

Eine Lösung von 89,4 g (0,24 Mol) 2-/4-(4-Chlor-2-trifluormethyl-phenoxy)-phenoxy/-propionsäure-methylester in 50 ml Methanol wird bei 40°C unter Rühren mit einem Gemisch aus 10,5 g (0,26 Mol) Natriumhydroxid, 100 ml Wasser und 50 ml Methanol versetzt. Man rührt weitere 16 Stunden bei 40°C und arbeitet dann auf, indem man das Lösungsmittel unter vermindertem Druck abzieht, den verbleibenden Rückstand in Wasser gibt und mit verdünnter wäßriger Salzsäure ansäuert. Das entstehende Gemisch wird mehrfach mit Chloroform extrahiert. Die vereinigten organischen Phasen werden getrocknet, unter vermindertem Druck eingeengt und anschließend durch leichtes Erwärmen im Hochvakuum von Resten an flüchtigen Anteilen befreit. Man erhält auf diese Weise 72,5 g (84,2 % der Theorie) an 2-/4-(4-Chlor-2-trifluormethyl-phenoxy)-phenoxy/-propionsäure in Form einer Festsubstanz vom Schmelzpunkt 60°C.

Beispiel 3

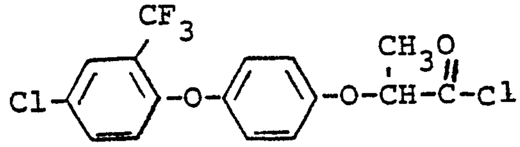

Zu einer Lösung von 252,4 g (0,71 Mol) 2-/4-(4-Chlor-2-trifluormethyl-phenoxy)-phenoxy7-propionsäure und 0,5 ml Dimethylformamid in 500 ml Methylenchlorid werden bei Rückflußtemperatur 100 g (0,84 Mol) Thionylchlorid zugetropft. Danach wird weitere 4 Stunden unter Rückfluß gerührt und dann aufgearbeitet, indem man das Lösungsmittel unter vermindertem Druck abzieht. Man erhält auf diese Weise 262 g (98,7 % der Theorie) an 2-/4-(4-Chlor-2-trifluormethyl-phenoxy)-phenoxy7-propionsäurechlorid in Form eines gelben Öles mit einem Brechungsindex von $n_D^{20}$ = 1,537.

Beispiel 4

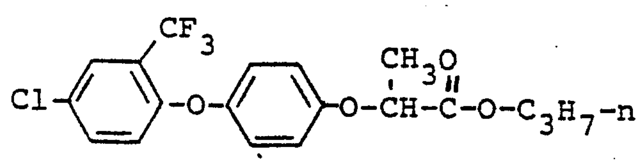

Zu einer Lösung von 1,5 g (0,025 Mol) n-Propanol und 2,8 g (0,028 Mol) Triethylamin in 50 ml Toluol werden bei 0°C unter Rühren 9,5 g (0,025 Mol) 2-/4-(4-Chlor-2-trifluormethylphenoxy)-phenoxy7-propionsäurechlorid hinzugetropft. Danach rührt man weitere 3 Stunden bei 20°C und arbeitet dann auf, indem man mit Wasser versetzt, die organische Phase abtrennt und diese nacheinander mit verdünnter wäßriger Salzsäure und mit Wasser wäscht. Nach dem Trocknen wird das Lösungsmittel unter vermindertem Druck abgezogen. Man erhält auf diese Weise 6,5 g (64 % der Theorie) an 2-/4-(4-Chlor-2-trifluormethyl-phenoxy)-phenoxy7-propionsäure-n-propylester in Form eines Öles

mit einem Brechungsindex von $n_D^{22}$ = 1,5164.

Nach den in den vorausgehenden Beispielen angegebenen Methoden werden auch die in der folgenden Tabelle 4 formelmäßig aufgeführten Stoffe erhalten.

Tabelle 4

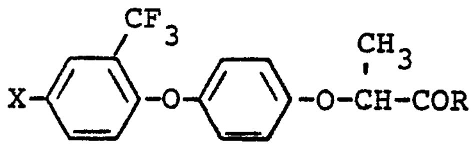

(I)

| Beispiel Nr. | X | R | Brechungsindex |
|---|---|---|---|
| 5 | Cl | $-O-CH_2-CF_3$ | $n_D^{22}$ = 1,4925 |
| 6 | Cl | $-O-CH_2-CCl_3$ | $n_D^{22}$ = 1,5323 |
| 7 | Cl | $-O-C_3H_7$-iso | $n_D^{22}$ = 1,5154 |
| 8 | Cl | $-O-CH_2-CH_2Cl$ | $n_D^{22}$ = 1,5292 |
| 9 | Cl | $-O-C_4H_9$-n | $n_D^{22}$ = 1,5185 |
| 10 | Cl | $-O-C_2H_5$ | $n_D^{22}$ = 1,5175 |
| 11 | Cl | $-O-C_4H_9$-iso | $n_D^{22}$ = 1,5144 |
| 12 | Cl | $-O-C_6H_{13}$-n | $n_D^{21,5}$ = 1,5095 |
| 13 | Cl | $-O-CH_2-CH=CH_2$ | $n_D^{21,5}$ = 1,5246 |
| 14 | Cl | $-O-CH(CH_2Cl)_2$ | $n_D^{21,5}$ = 1,5298 |
| 15 | Cl | $-O-CH_2-C\equiv CH$ | $n_D^{21,5}$ = 1,5285 |
| 16 | Cl | -O-CH- 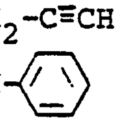 $CH_3$ | $n_D^{21,5}$ = 1,5405 |
| 17 | Cl | $-O-C_2H_4-$ 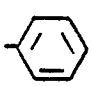 | $n_D^{21,5}$ = 1,5433 |
| 18 | Cl | $-O-CH_2-CH_2-OCH_3$ | $n_D^{21,5}$ = 1,5192 |
| 19 | Cl | $-S-C_2H_5$ | $n_D^{21,5}$ = 1,5437 |

Tabelle 4 (Fortsetzung)

| Beispiel-Nr. | X | R | Berechnungsindex bzw. Schmelzpunkt (Fp) |
|---|---|---|---|
| 20 | H | $-OC_2H_5$ | $n_D^{21}$ = 1,5078 |
| 21 | H | $-OC_3H_7$-iso | $n_D^{21}$ = 1,5031 |
| 22 | H | $-OCH_2CF_3$ | $n_D^{21}$ = 1,4795 |
| 23 | H | $-OC_4H_9$-iso | $n_D^{21}$ = 1,5010 |
| 24 | H | $-O-CH(CH_3)-C{\equiv}CH$ | $n_D^{21,5}$ = 1,5107 |
| 25 | H | $-O-CH_2-$ 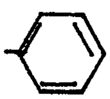 | $n_D^{21,5}$ = 1,5396 |
| 26 | Cl | $-S-C_4H_9$-n | $n_D^{21,5}$ = 1,5357 |
| 27 | Cl | $-O-CH_2-$ 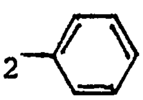 | $n_D^{23}$ = 1,5467 |
| 28 | Cl | $-O-C_2H_4-S-C_2H_5$ | $n_D^{23}$ = 1,5315 |
| 29 | Cl | $-O-CH(CH_3)-C{\equiv}CH$ | $n_D^{21,5}$ = 1,5205 |
| 30 | H | $-OCH_3$ | $n_D^{21}$ = 1,5142 |
| 31 | H | -OH | Fp = 116°C |

Beispiel 32

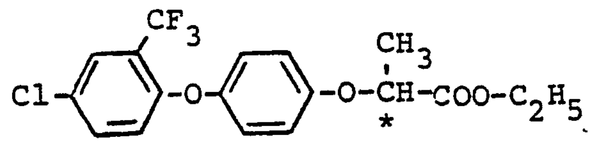 R-Isomeres

Ein Gemisch aus 28,8 g (0,1 Mol) 4-(4-Chlor-2-trifluormethyl-phenoxy)-phenol, 27,2 g (0,1 Mol) S-(-)-Milchsäureethylester-tosylat und 16,6 g (0,12 Mol) wasserfreiem Kaliumcarbonat in 200 ml Acetonitril wird 15 Stunden unter Rückfluß erhitzt. Dann wird das Reaktionsgemisch abgekühlt und in 400 ml Wasser gegeben. Es wird zweimal mit je 200 ml Toluol extrahiert. Die Toluolphase wird über Natriumsulfat getrocknet und dann eingeengt. Man erhält 34 g (87,5 % der Theorie) R-2-/4-(4-Chlor-2-trifluormethyl-phenoxy)-phenoxy7-propionsäureethylester in Form eines gelben Öles mit dem Brechungsindex $n_D^{19}$ = 1,518. Drehwert: $[\alpha]_D^{24}$ = + 6,2° (1-molare Lösung in Chloroform; Küvettenlänge 10 cm)

Beispiel 33

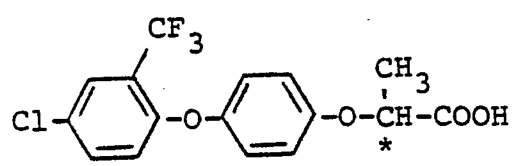 R-Isomeres

Zu einer intensiv gerührten Mischung von 38,8 g (0,01 Mol) R-2-/4-(4-Chlor-2-trifluormethylphenoxy)-phenoxy7-propionsäureethylester in 150 ml Xylol werden bei 110°C 8 g (0,2 Mol) Natriumhydroxid in 50 ml Wasser getropft. Man rührt 15 Minuten bei 110°C nach, kühlt ab und versetzt das Reaktionsgemisch mit 200 ml Wasser.

Die wäßrige Phase wird abgetrennt, mit konzentrierter, wäßriger Salzsäure angesäuert und zweimal mit je 300 ml Ether extrahiert. Die vereinigten Etherextrahte werden über Natriumsulfat getrocknet und dann eingeengt. Man erhält 22 g (61 % der Theorie) R-2-[4-(4-Chlor-2-trifluormethylphenoxy)-phenoxy]-propionsäure. Drehwert: $[\alpha]_D^{24}$ = + 3,6°
(1-molare Lösung in Chloroform; Küvettenlänge 10 cm)

<u>Beispiel 34</u>

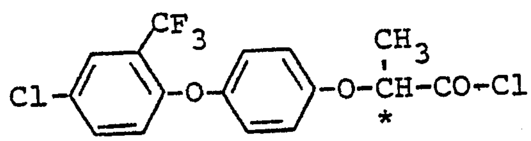 R-Isomeres

Zu einer Lösung von 36 g (0,1 Mol) R-2-[4-(4-Chlor-2-trifluormethylphenoxy]-phenoxy-propionsäure in 200 ml Toluol werden 15,3 g (0,13 Mol) Thionylchlorid getropft. Man erhitzt 10 Stunden zum Sieden und destilliert dann das Lösungsmittel ab. Man erhält 34,8 g (92 % der Theorie) R-2-[4-(4-Chlor-2-trifluormethylphenoxy)-phenoxy]-propionsäurechlorid in Form eines gelben Öles vom Brechungsindex $n_D^{20}$ = 1,536.

Herstellung von Ausgangsprodukten

Beispiel 35

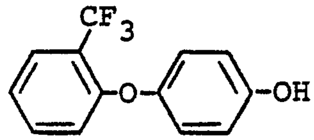

(II-2)

In eine Mischung aus 50 ml Dimethylsulfoxid, 13,2 g (0,12 Mol) Hydrochinon und 11,4 g gepulvertem Kaliumhydroxid wird bei einer Temperatur von 80°C bis 90°C unter Rühren innerhalb einer Stunde eine Lösung von 22,5 g (0,1 Mol) 2-Brom-benzotrifluorid in 20 ml Dimethylsulfoxid getropft. Man rührt weitere 4 Stunden bei 80°C und zieht dann das Lösungsmittel ab. Der verbleibende Rückstand wird in 150 ml Wasser gegossen, und die entstehende Mischung wird mit 10 ml konzentrierter wäßriger Salzsäure angesäuert. Das Gemisch wird zweimal mit je 100 ml Chloroform extrahiert. Die vereinigten organischen Phasen werden zweimal mit einer Lösung von 100 ml Wasser und 5 ml konzentrierter wäßriger Natronlauge ausgeschüttelt. Man säuert die vereinigten wäßrigen Phasen mit 15 ml konzentrierter wäßriger Salzsäure an und extrahiert das sich dabei abscheidende Öl zweimal mit je 100 ml Chloroform. Man engt die vereinigten organischen Phasen ein und erhält auf diese Weise 15 g (65,2 % der Theorie) an 2-Trifluormethyl-4'-hydroxy-diphenylether in Form eines Öles mit dem Brechungsindex $n_D^{20}$ = 1,5231.

Siedepunkt: 121°C / 0,5 mbar.

Beispiel 36

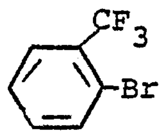

(VII-2)

(Variante δ)

Auf ein Gemisch von 200 g (1,27 Mol) Brombenzol in 500 ml Tetrachlorkohlenstoff läßt man 500 g Fluorwasserstoff in einer Fluorierungsapparatur für 10 Stunden bei 108°C bis 110°C einwirken. Der entstehende Chlorwasserstoff wird entspannt. Nach Beendigung der Umsetzung arbeitet man auf, indem man die organische Phase destilliert. Man erhält auf diese Weise 51 g (17,8 % der Theorie) an 2-Brom-benzotrifluorid. Kp = 60 - 63°C/18 mbar

$n_D^{20}$ = 1,4810.

In dem nachfolgend beschriebenen biologischen Test wurde die folgende Verbindung als Vergleichssubstanz eingesetzt:

(A) =

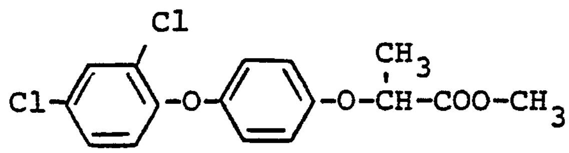

2-/4-(2,4-Dichlorphenoxy)-phenoxy7-propionsäure-methylester (bekannt aus DE-OS 2 223 894).

Beispiel A

Post-emergence-Test

Lösungsmittel: 5 Gewichtsteile Aceton
Emulgator: 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeutet:

0 % = keine Wirkung (wie unbehandelte Kontrolle)
100 % = totale Vernichtung

In diesem Test zeigt der erfindungsgemäße Wirkstoff (1) eine bessere selektive herbizide Wirksamkeit als die Vergleichssubstanz (A).

Patentansprüche

1) Phenoxypropionsäure-Derivate der Formel

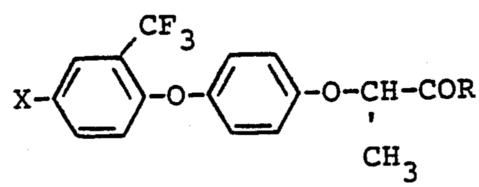

in welcher

R für Hydroxy, Chlor, Alkoxy, Halogenalkoxy, Alkoxyalkoxy, Alkenoxy, Alkinoxy, Aralkoxy, Alkylthio oder Alkylthioalkoxy steht und

X für Wasserstoff oder Halogen steht.

2) Phenoxypropionsäure-Derivate der Formel (I), in denen R für Hydroxy, Chlor, Alkoxy mit 1 bis 8 Kohlenstoffatomen, Halogenalkoxy mit 1 bis 8 Kohlenstoffatomen und 1 bis 5 Halogenatomen, Alkoxyalkoxy mit 1 bis 6 Kohlenstoffatomen in jeder Alkoxygruppe, Alkenoxy mit 2 bis 6 Kohlenstoffatomen, Alkinoxy mit 2 bis 6 Kohlenstoffatomen, Aralkoxy mit 6 bis 10 Kohlenstoffatomen im Arylteil und 1 bis 4 Kohlenstoffatomen im Alkoxyteil, für Alkylthio mit 1 bis 6 Kohlenstoffatomen sowie für Alkylthioalkoxy mit 1 bis 6 Kohlenstoffatomen in der Alkylthiogruppe und 1 bis 6 Kohlenstoffatomen im Alkoxyteil steht und X für Fluor, Chlor oder Brom steht.

3) Verfahren zur Herstellung von Phenoxypropionsäure-Derivaten der Formel

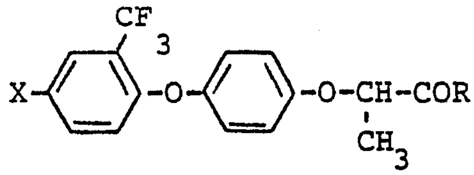

(I)

in welcher

R für Hydroxy, Chlor, Alkoxy, Halogenalkoxy, Alkoxyalkoxy, Alkenoxy, Alkinoxy, Aralkoxy, Alkylthio oder Alkylthioalkoxy steht und

X für Wasserstoff oder Halogen steht,

dadurch gekennzeichnet, daß man

a) Diphenylether der Formel

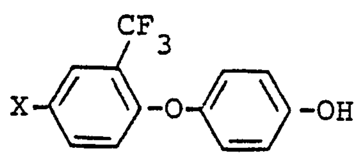

(II)

in welcher

X die oben angegebene Bedeutung hat

mit Propionsäure-Derivaten der Formel

$$Y - \underset{\displaystyle CH_3}{\underset{|}{CH}} - COR^1 \qquad (III)$$

in welcher

$R^1$ für Alkoxy, Halogenalkoxy, Alkoxyalkoxy, Alkenoxy, Alkinoxy, Aralkoxy, Alkylthio oder Alkylthioalkoxy steht und

Y für Halogen, Tosylat oder Mesylat steht,

gegebenenfalls in Gegenwart eines Säureakzeptors und gegebenenfalls in Gegenwart eines Verdünnungsmittels umsetzt,

oder

b) Phenoxypropionsäure-Derivate der Formel

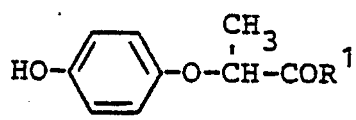

(IV)

in welcher

$R^1$ die oben angegebene Bedeutung hat,

mit Trifluormethylbenzol-Derivaten der Formel

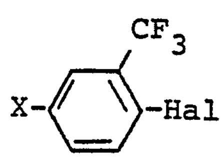

(V)

in welcher

X die oben angegebene Bedeuutng hat und

Hal für Chlor oder Brom steht,

gegebenenfalls in Gegenwart eines Säureakzeptors und gegebenenfalls in Gegenwart eines Verdünnungsmittels umsetzt,

oder

c) Phenoxypropionsäure-Derivate der Formel

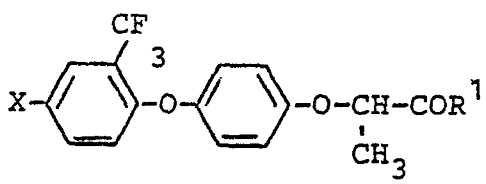

(Ia)

in welcher

$R^1$ und X die oben angegebene Bedeutung haben,

mit starken Basen in Gegenwart eines Verdünnungsmittels behandelt und anschließend ansäuert,

oder

d) Phenoxypropionsäure-Derivate der Formel

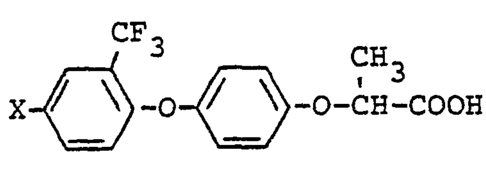

(Ib)

in welcher

X die oben angegebene Bedeutung hat,

mit Thionylchlorid gegebenenfalls in Gegenwart eines Verdünnungsmittels sowie gegebenenfalls in Gegenwart eines Katalysators umsetzt,

oder

e) Phenoxypropionsäure-Derivate der Formel

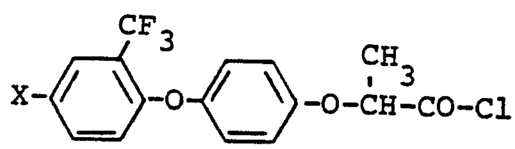

(Ic)

in welcher

X die oben angegebene Bedeutung hat,

mit Verbindungen der Formel

$$MR^1 \qquad (VI)$$

in welcher

$R^1$ die oben angegebene Bedeutung hat und

M für Wasserstoff, Natrium oder Kalium steht,

gegebenenfalls in Gegenwart eines Säurebindemittels und gegebenenfalls in Gegenwart eines Verdünnungsmittels umsetzt.

4) Herbizide Mittel, gekennzeichnet, durch einen Gehalt an mindestens einem Phenoxypropionsäure-Derivat der Formel (I).

5) Verfahren zur Bekämpfung von Unkräutern, dadurch gekennzeichnet, daß man Phenoxypropionsäure-Derivate der Formel (I) auf die Unkräuter und/oder deren Lebensraum ausbringt.

6) Verwendung von Phenoxypropionsäure-Derivaten der Formel (I) zur Bekämpfung von Unkräutern.

7) Verfahren zur Herstellung von herbiziden Mitteln, dadurch gekennzeichnet, daß man Phenoxypropionsäure-Derivate der Formel (I) mit Streckmitteln und/oder oberflächenaktiven Stoffen vermischt.

8) Diphenylether der Formel

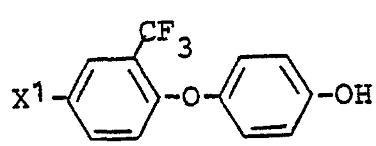

(IIa)

in welcher

$X^1$ für Wasserstoff, Fluor oder Brom steht.

9) Verfahren zur Herstellung von Diphenylethern der Formel

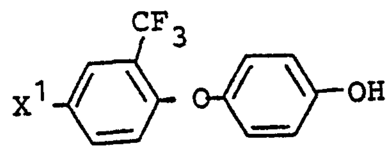

(IIa)

in welcher

$X^1$ für Wasserstoff, Fluor oder Brom steht,

dadurch gekennzeichnet, daß man Trifluormethylbenzol-Derivate der Formel

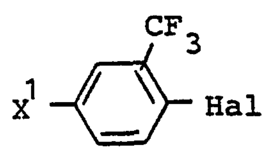

(Va)

in welcher

$X^1$ die oben angegebene Bedeutung hat und

Hal für Chlor oder Brom steht,

mit Hydrochinon in Gegenwart eines Säureakzeptors sowie in Gegenwart eines polaren Verdünnungsmittels umsetzt.

10) R-Enantiomere von Phenoxypropionsäure-Derivaten der Formel (I), in denen
R für Hydroxy, Chlor, Alkoxy mit 1 bis 8 Kohlenstoffatomen, Halogenalkoxy mit 1 bis 8 Kohlenstoffatomen und 1 bis 5 Halogenatomen, Alkoxyalkoxy mit 1 bis 6

Kohlenstoffatomen in jeder Alkoxygruppe, Alkenoxy mit 2 bis 6 Kohlenstoffatomen, Alkinoxy mit 2 bis 6 Kohlenstoffatomen, Aralkoxy mit 6 bis 10 Kohlenstoffatomen im Arylteil und 1 bis 4 Kohlenstoffatomen im Alkoxyteil, für Alkylthio mit 1 bis 6 Kohlenstoffatomen sowie für Alkylthioalkoxy mit 1 bis 6 Kohlenstoffatomen in der Alkylthiogruppe und 1 bis 6 Kohlenstoffatomen im Alkoxyteil steht und X für Fluor, Chlor oder Brom oder Wasserstoff steht.

Europäisches Patentamt

# EUROPÄISCHER RECHERCHENBERICHT

0095117
Nummer der Anmeldung

EP 83104792.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | DE - A1 - 2 609 461 (HOECHST AG)<br>* Seiten 3-8 *<br>-- | 1,3-7 | C 07 C 59/68<br>C 07 C 69/712<br>C 07 C 149/14<br>C 07 C 51/00<br>C 07 C 67/00<br>C 07 C 43/295<br>C 07 C 41/16<br>C 07 C 153/07<br>A 01 N 39/04 |
| X | EP - A1 - 0 003 562 (BAYER AKTIEN-GESELLSCHAFT)<br>* Ansprüche *<br>-- | 8,9 | |
| A | DE - A1 - 2 805 982 (BAYER AG)<br>* Ansprüche *<br>-- | 1,3-7 | |
| A | DE - A1 - 2 946 652 (SHELL INTERNATIONALE RESEARCH MAATSCHAPPIJ B.V.)<br>* Ansprüche 1,6-11 *<br>---- | 1,3-7, 10 | RECHERCHIERTE SACHGEBIETE (Int. Cl. 3)<br>C 07 C 43/00<br>C 07 C 59/00<br>C 07 C 69/00<br>C 07 C 149/00 |

*Der vorliegende Recherchenbericht wurde für alle Patentansprüche erstellt.*

| Recherchenort | Abschlußdatum der Recherche | Prüfer |
|---|---|---|
| WIEN | 18-07-1983 | HOFBAUER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument